# EUROPÄISCHE PATENTSCHRIFT

(11) **EP 1 200 026 B1**
(45) Veröffentlichungstag und Bekanntmachung des Hinweises auf die Patenterteilung: **07.09.2005**
(21) Anmeldenummer: 01911483.4
(22) Anmeldetag: 15.01.2001
(51) Int. Cl.: A61F 9/01

(54) **VORRICHTUNG FÜR DIE OPHTALMOLOGISCHE AUGENBEHANDLUNG MIT FIXIERLICHTSTRAHL**
DEVICE FOR OPHTHALMOLOGICALLY TREATING THE EYE USING A FIXATION LIGHT BEAM
DISPOSITIF DE TRAITEMENT OPHTALMOLOGIQUE DES YEUX PAR FAISCEAU LUMINEUX DE FIXATION

(30) Priorität: 13.01.2000 DE 10001131
(43) Veröffentlichungstag der Anmeldung: 02.05.2002
(73) Patentinhaber: WaveLight Laser Technologie AG, 91058 Erlangen (DE)
(72) Erfinder: DONITZKY, Christof, 90542 Eckental (DE)
(74) Vertreter: von Hellfeld, Axel
(86) Internationale Anmeldenummer: PCT/EP2001/000393
(87) Internationale Veröffentlichungsnummer: WO 2001/050990

(56) Entgegenhaltungen:
- EP-A- 0 770 370
- DE-A- 19 618 883
- US-A- 4 443 075
- US-A- 5 029 220
- US-A- 5 644 642

## Beschreibung

Die Erfindung betrifft eine Vorrichtung für die ophtalmologische Augenbehandlung mit zumindest einem Behandlungslaserstrahl zum Ablatieren von Teilen der Kornea und einem Fixierlichtstrahl, der vorgesehen ist, um vom Patienten fixiert zu werden.

Bei der photorefraktiven Keratektomie (PRK) (englisch: photorefractive keratectomy) wird eine Fehlsichtigkeit des menschlichen Auges dadurch korrigiert, daß die Kornea teilweise neu geformt wird. Ein besonderes, zur Zeit erheblich an Bedeutung gewinnendes Verfahren der PRK ist LASIK. Bei dem LASIK-Verfahren wird ein Deckel ("Flap") in die Kornea geschnitten und aufgeklappt. Sodann wird mit dem UV-Laserstrahl (üblicherweise ein Excimer-Laserstrahl mit einer Wellenlänge von 193 nm) auf die freiliegenden (durch den Deckel freigegebenen) Teile der Kornea gerichtet, um dort Material abzutragen (zu ablatieren). Nach der gewünschten Ablation wird der Dekkel wieder zugeklappt und verheilt mit der Kornea.

Die vorliegende Erfindung betrifft allgemein PRK und insbesondere das LASIK-Verfahren.

Bei den photorefraktiven ophtalmologischen Verfahren kommt es darauf an, das Auge in bezug auf die verwendete Laserstrahlung, insbesondere den Ablationsstrahl, genau zu positionieren, d. h. bei jedem Laserpuls, der ablatierend auf das Auge auftrifft, muß das System genau "wissen", an welcher Stelle des Auges der Laserstrahl auftrifft. Hierzu werden im Stand der Technik sog. "Eye-Tracker" eingesetzt. Dies sind Vorrichtungen, mit denen die jeweilige momentane Position des Auges ermittelt wird, um entsprechend dieser ermittelten Position den Laserstrahl zu steuern. Der Laserstrahl wird dabei z. B. mittels eines Scanners zeitlich und örtlich gesteuert über die zu bearbeitende Augenfläche (bei LASIK z. B. im Stroma) geführt, wobei die zeitliche und örtliche Steuerung des Laserspots (fokussierter Fleck) so ist, daß ein gewünschtes Ablationsprofil abgetragen (ablatiert) wird. Es ist dabei Stand der Technik, einen sog. Fixierlichtstrahl einzusetzen. Eine Fixierlichtquelle wird so positioniert, daß der Patient sie visuell fixieren kann. Hierzu wird der Patient aufgefordert. Dies hat zum Ziel, das Auge möglichst konstant dadurch stillzusetzen, daß der Patient ununterbrochen die Fixierlichtquelle fixiert. Da der Patient die Fixierlichtquelle dabei erkennen muß, versteht sich, daß die Fixierlichtquelle einen Fixierlichtstrahl mit Wellenlängen im sichtbaren Bereich abgibt, z. B. im grünen Bereich.

Allerdings gelingt es den Patienten in aller Regel nicht, den Fixierlichtstrahl völlig ununterbrochen zu fixieren. Im Stand der Technik sind deshalb die genannten "Eye-Tracker" bekannt, d. h. opto-elektronische Systeme, mit denen Bewegungen des Auges erfaßt werden. Diese Bewegungen treten dann auf, wenn der Patient (ungewollt) für mehr oder weniger kurze Zeitspannen die Fixierlichtquelle im Wortsinne "aus dem Auge verliert". Fixiert der Patient die Fixierlichtquelle in idealer Weise, wird das Fixierlicht genau auf die Fovea abgebildet. Verliert der Patient hingegen die Fixierlichtquelle aus dem Auge, wird das Fixierlicht nicht mehr auf der Fovea abgebildet, sondern auf einer anderen Stelle der Netzhaut, also neben der Fovea, mehr oder weniger weit von ihr entfernt. Die genannten Eye-Tracker des Standes der Technik, der hier als bekannt vorausgesetzt wird, ermitteln z. B. Bewegungen des Auges durch Aufnehmen der Pupille mittels einer Kamera und einer Bildauswertung, bei der Bewegungen der Pupille festgestellt werden. Der Behandlungslaserstrahl wird dann so gesteuert, daß derart festgestellte Augenbewegungen berücksichtigt werden und die Ablation trotz der Augenbewegungen genau entsprechend dem gewünschten Ablationsprofil erfolgt.

US-5,644,642 offenbart ein "Eye Tracking"- System, bei dem Reflexionen eines Fixierlichtstrahls auf der Kornea, insbesondere im Bereich der Iris ausgewertet werden, um auf Bewegungen eines Auges zu schließen.

EP 0 770 370 A2 offenbart eine Vorrichtung zum Entfernen von Korneagewebe, gemäß dem Oberbegriff des ersten Anspruchs, mit Infrarotlaserstrahlung, die einen "Eye Tracker" umfasst. Der "Eye Tracker" erfasst laterale Bewegungen eines Auges, indem ein optisches Bild eines signifikanten Merkmal des Auges erzeugt wird. Insbesondere wird als signifikantes Augenmerkmal der kreisförmige Schnittbereich der transparenten Kornea mit der lichtdurchlässig und weißgefärbte Sklera verwendet.

Der vorliegenden Erfindung liegt die Erkenntnis zugrunde, daß es bei der relativer Positionierung von Auge-und Ablationslaserstrahl bei Verwendung von Fixierlicht zu besonderen Problemen dadurch kommen kann, daß der Patient im Streß der Operation oder aus anderen Gründen die Fixierlichtquelle überhaupt nicht korrekt fixiert. Die Erfindung hat das Ziel, in einer derartigen Situation Abhilfe zu schaffen.

Erfindungsgemäß wird dieses Ziel durch eine Kamera erreicht, mit der das Abbild des Fixierlichtstrahls auf der Netzhaut, insbesondere im Bereich der Fovea, und die Fovea aufgenommen werden. Im Idealfall liegt nämlich der Fleck (Spot) des Fixierlichts genau auf der Fovea, wenn der Patient die Fixierlichtquelle korrekt fixiert. Fixiert der Patient hingegen die Fixierlichtquelle nicht korrekt, wird das Fixierlicht nicht genau auf der Fovea abgebildet, sondern an einer anderen Stelle der Netzhaut. Dies kann dann mit der erfindungsgemäß vorgesehenen Kamera erkannt werden. Die optischen Abbildungselemente dieser Kamera sind also so ausgelegt, daß mit der Kamera Bilder in der Netzhautebene im Bereich der Fovea scharf abgebildet werden. Somit kann der Augenarzt vor und während der Operation feststellen, ob der Patient die Fixierlichtquelle vorschriftsmäßig fixiert "im Auge hat". Diese Überprüfung kann auch durch Bildverarbeitung automatisiert werden. Dabei wird das Kamerasystem auf die Netzhautfläche in Höhe der Fovea zumindest annähernd scharf gestellt und ein Bild der Fovea und der benachbarten Bereiche der Netzhaut aufgenommen. Mittels Bildverarbeitung kann dann festgestellt werden, ob der Spot des Fixierlichtes hinreichend genau auf der Fovea liegt oder nicht. Wird festgestellt, daß der Patient die Fixierlichtquelle nicht hinreichend genau, d. h. mit hinreichend wenig Abweichungen (zeitlich und abstandsmäßig gesehen) fixiert, kann der Arzt daraus für die Behandlung Schlußfolgerungen ziehen und gegebenenfalls Maßnahmen treffen. Die Überprüfung der relativen Lage des Fixierlicht-Lichtfleckes (Spot) auf der Netzhaut in bezug auf die Fovea kann vor und/oder während der Operation erfolgen.

Gemäß einer bevorzugten Ausgestaltung der Erfindung wird nicht nur die Lage des Fixierlicht-Spots in bezug auf die Fovea gemessen und ausgewertet, sondern auch zusätzlich die Pupille mit einer Kamera aufgenommen. Beide Aufnahmen, also die Aufnahme von Fixierlicht-Spot und Fovea einerseits und die Aufnahme der Pupille andererseits werden in bezug auf eine gemeinsame, festbleibende Achse durchgeführt, z. B. die Fixierlichtachse. Die beiden Bilder unterscheiden sich also durch die Fokusebene: Die Abbildung der Pupille ist in bezug auf die Abbildung von Fixierlicht-Spot und Fovea um etwa den Augendurchmesser versetzt, also 2 bis 3 cm.

Die Aufnahme von Pupille und Fovea mit Fixierlicht-Spot können bevorzugt einander überlagert werden, d. h. es entsteht ein Bild, auf dem zum einen die Pupille abgebildet ist und zum anderen die Fovea und der Fixierlicht-Spot. Dies ermöglicht nicht nur eine Überprüfung, ob der Patient die Fixierlichtquelle zuverlässig fixiert, sondern auch eine Festlegung einer günstigen Zentralachse für die Ablation. Fixiert der Patient die Fixierlichtquelle, dann erscheinen auf dem vorstehend erläuterten überlagerten Bild die Pupille, die Fovea (genauer: die Macula Lutea) und der Fixierlicht-Spot, letzterer genau zentrisch in der Fovea. Bei idealen Verhältnissen liegt der Fixierlicht-Spot (und die Fovea) zentrisch in der Pupille. Bei realen Verhältnissen ist jedoch häufig der Fixierlicht-Spot mit der Fovea nicht zentrisch in bezug auf die Pupille, d. h. auf dem überlagerten Bild ist der Fixierlicht-Spot mit der Fovea in bezug auf das Pupillenzentrum versetzt. Bei diesem Befund erfolgt die nachfolgende Ablation gemäß einer bevorzugten Ausgestaltung der Erfindung zentriert in bezug auf einen Punkt, der exzentrisch in bezug auf die Pupille ist und der durch den Mittelpunkt von Fixierlicht-Spot und Fovea definiert ist (bei genauer Fixierung ist dies ein und derselbe Punkt).

Die beiden vorstehend genannten Aufnahmen, also Pupille einerseits und Fixierlicht-Spot mit Fovea andererseits, können durch eine einzige Kamera erzeugt werden, wenn die Abbildungsebene (Fokus) für die beiden Aufnahmen in der Art eines Autofokus-Effektes variiert wird. Dies kann mit geeigneten Zoom-Mitteln periodisch in kurzer Zeitfolge geschehen, so daß das genannte Überlagerungsbild erzeugt werden kann.

Es ist andererseits aber auch möglich, und wird zur Zeit bevorzugt, mit zwei Kameras zu arbeiten, d. h. eine erste Kamera nimmt die Pupille auf und eine andere Kamera gleichzeitig die Fovea mit dem Fixierlicht-Spot. In einem Rechner können beide Bilder zusammengeführt und überlagert werden, sowie auf einem Display im überlagerten Zustand angezeigt werden bzw. in der oben beschriebenen Weise einer automatisierten Bildverarbeitung zugeführt werden.

Die verwendeten Kameras sind bevorzugt Videokameras, besonders bevorzugt Festkörper-Videokameras z. B. CCD oder dergleichen.

Bevorzugt kann dabei eine Kamera verwendet werden, die im ophtalmologischen Behandlungssystem sowieso für die Zwecke des "Eye-Tracking" vorhanden ist.

Nachfolgend wird ein Ausführungsbeispiel der Erfindung anhand der Zeichnung näher erläutert. Es zeigt bzw. zeigen:
- Figur 1: schematisch eine Vorrichtung für die photorefraktive Keratektomie des Auges, insbesondere gemäß dem LASIK-Verfahren; und
- Figuren 2, 3 und 4: schematisch Überlagerungsbilder von Pupille, Fovea und Fixierlicht-Spot in unterschiedlichen Situationen.

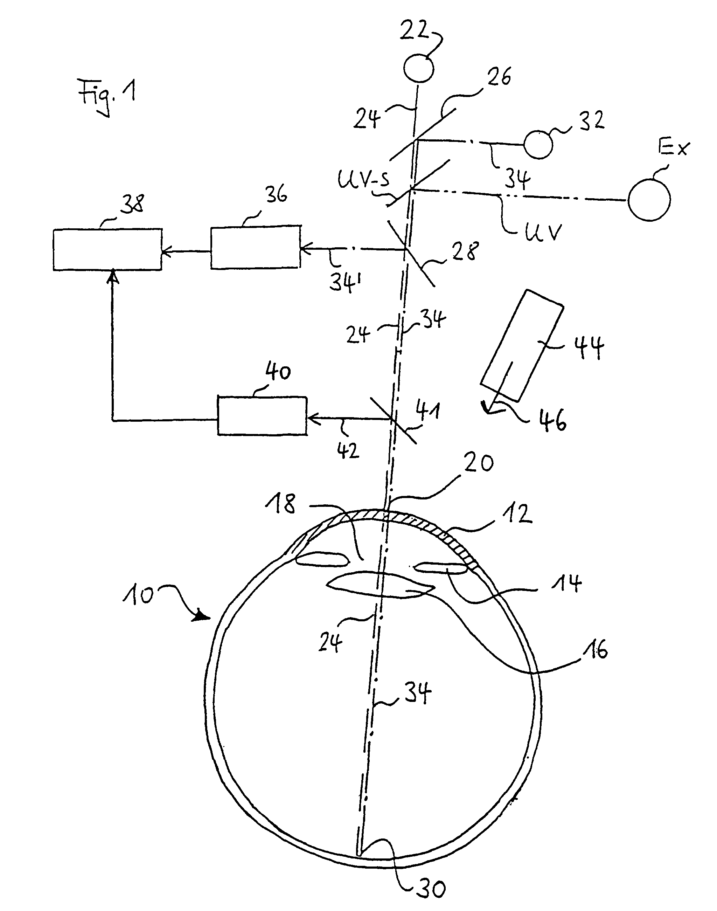

Das in Figur 1 schematisch gezeigte Auge 10 hat eine Kornea 12, eine Iris 14, eine Linse 16 und eine Pupille 18.

Eine als solches bekannte Fixierlichtquelle 22 gibt einen Fixierlichtstrahl 24 ab, der an der Stelle 20 die Vorderfläche der Kornea 12 durchstößt. Die Wellenlänge des Fixierlichtstrahls 24 ist so, daß sie für den Patienten sichtbar ist, liegt also z. B. im grünen Bereich des Spektrums. Als Fixierlichtquelle 22 wird üblicherweise eine Diode verwendet. Der Fixierlichtstrahl 24 ist ortsfest und der Patient ist angehalten, die ihm punktförmig erscheinende Fixierlichtquelle zu fixieren.

Ein Excimerlaser Ex emittiert den eigentlichen Ablationsstrahl, also den Strahl, mit dem die Kornea 12 neu geformt wird. Dieser Ablationsstrahl UV (z.B. 193 nm) wird über einen Spiegel UV-S umgelenkt und gemäß einem Ablationsalgorithmus über die Kornea 12 geführt, so daß das gewünschte Ablationsprofil abgetragen wird. Der Ablationsstrahl ist also nicht ortsfest. Die Mittel zum Bewegen ("scannen") des Ablationsstrahls sind als solches bekannt und in der Figur nicht näher dargestellt.

Der Fixierlichtstrahl 24 tritt durch die Kornea und die Pupille 18 und wird auf die Fovea 30 abgebildet. Er wird deshalb auch als "Gesichtslinie" ("line of sight") bezeichnet. Diese Gesichtslinie verbindet also objektseitig den Fixationspunkt (also den Punkt der Fixierlichtquelle 22) mit dem Zentrum der Eintrittspupille. Die "Eintrittspupille" ist das virtuelle Bild der realen Pupille, das ein Betrachter beim Blick auf das Auge sieht.

Die Stelle 20, an der der Fixierlichtstrahl 24 die Vorderfläche der Kornea 12 durchstößt, kann als Zentrum für die Ablation gewählt werden, d. h. das Ablationsprofil, gemäß dem der Ablationsstrahl UV über die Kornea 12 geführt ("gescannt") wird, wird auf den Punkt 20 zentriert, in dem der Fixierlichtstrahl 24 die freiliegende Vorderfläche der Kornea 12 durchstößt. Beim LASIK-Verfahren ist die Vorderfläche der Kornea in diesem Sinne die freiliegende Fläche, nach Aufklappen des sog. Deckels (Flap). Um den Durchstoßungspunkt 20 auf der Kornea 12 zu ermitteln, wird eine Zentrierlichtquelle 32 verwendet, die beim dargestellten Ausführungsbeispiel einen Laserstrahl im Infrarotbereich emittiert. Dieser Zentrierlichtstrahl 34 wird über einen teildurchlässigen Spiegel 26 koaxial mit dem Fixierlichtstrahl 24 auf die Kornea 12 gerichtet. In der Figur sind der Fixierlichtstrahl 24 und der Zentrierlichtstrahl 34 parallel nebeneinander gezeichnet, jedoch verlaufen sie tatsächlich koaxial, d. h. auf einer gemeinsamen Zentralachse. Dies bedeutet, daß der während der Operation ortsfeste Zentrierlichtstrahl 34 auch am Durchstoßungspunkt 20 durch die Vorderfläche der Kornea 12 tritt. Beim Ausführungsbeispiel hat der Zentrierlichtstrahl 34 eine Wellenlänge im infraroten Bereich, z. B. im Bereich von 800 bis 1100 nm. Wichtig ist, daß der Zentrierlichtstrahl 34 eine Wellenlänge hat, die verschieden ist von der Wellenlänge des Fixierlichtstrahls 24, so daß Reflexe und Bilder, die von beiden Strahlen erzeugt werden, voneinander diskriminiert werden können, d. h. aufgrund der unterschiedlichen Wellenlängen ist es möglich, einen Reflex des Zentrierlichtstrahls 34 auf der Vorderfläche der Kornea 12 ohne Störung durch den Fixierlichtstrahl zu vermessen. Dementsprechend wird der Durchstoßungspunkt 20 dadurch gemessen, daß der Streulicht-/Fresnelreflex des Zentrierlichtstrahls auf der Korneavorderfläche gemessen wird. Hierzu dient ein teildurchlässiger Spiegel 28, der den Streulicht-/Fresnelreflex 34' des Zentrierlichtstrahls auf eine Kamera 36 lenkt. Die Kamera 36 ist beim dargestellten Ausführungsbeispiel auch aus anderen Gründen Teil der Vorrichtung, nämlich als sog. "Eye-Tracking-Kamera" (vgl. DE 197 02 335 und den darin genannten Stand der Technik).

Die Verwendung eines besonderen Zentrierlichtstrahls 34 für die Bestimmung des Durchstoßungspunktes 20 der ortsfesten Strahlung auf der Korneavorderfläche hat im Vergleich zur Verwendung des Fixierlichtstrahls 24 hierfür den Vorteil, daß eine relativ lichtstarker und nicht von anderen Bildern überstrahlter Reflex mittels der Kamera 36 und eines nachgeschalteten Auswerterechners 38 ausgewertet werden kann. Auch wird der Streulicht-/Fresnelreflex des Fixierlichtes selbst vom Purkinje-Sanson Bild überstrahlt, so daß dieser Reflex schwer auszuwerten ist.

Die Kamera 36 und der Rechner 38, in den die Kamera-Meßwerte eingegeben werden, bilden ein sog. Eye-Tracking-System (vgl. den oben genannten Stand der Technik). Hierzu wird das Auge mit unabhängiger Strahlung, z.B. IR-Strahlung 46, erzeugt von einer Lichtquelle 44, beleuchtet und z. B. die Pupille 18 mittels ihres Randes vermessen, um insbesondere das geometrische Zentrum der Pupille (der sog. "Pupillenschwerpunkt") zu ermitteln. Daneben vermißt nun das System aus Kamera 36 und Rechner 38 auch die Position des Streulicht-/Fresnelreflexes des Zentrierlichtstrahls 34 auf der Vorderfläche der Kornea 12, d. h. an der Stelle des Durchstoßungspunktes 20. Die Kamera 36 ist somit beim dargestellten Ausführungsbeispiel IRempfindlich. Bevorzugt ermittelt das System aus Kamera 36 und Rechner 38 die relative Position zwischen Durchstoßungspunkt 20 und geometrischem Zentrum ("Schwerpunkt") der Pupille 18.

Um zu überprüfen, ob der Patient die Fixierlichtquelle 22 (d. h. den Fixierlichtstrahl 24) hinreichend exakt fixiert, ist beim Ausführungsbeispiel gemäß Figur 1 eine weitere Kamera 40 vorgesehen. Die Kamera 40 ist z. B. eine Videokamera (CCD) und die Bildsignale werden elektrisch ebenfalls in den Rechner 38 eingegeben. Die optischen Mittel (nicht gezeigt) der Kamera 40 sind so ausgelegt, daß sie ein Bild in der Ebene der Fovea 30 des Auges 10 aufnimmt. Der abgebildete Fleck (Spot) des Fixierlichtstrahls 24 wird im Idealfall, d. h. wenn der Patient die Fixierlichtquelle 22 genau fixiert, genau auf der Fovea 30 liegen. Ist die Fixierung seitens des Patienten ungenau, liegt der Fixierlicht-Spot neben der Fovea 30. Die Kamera 40 empfängt also über einen teildurchlässigen Spiegel 41 Strahlung, mit der die Bildebene der Fovea und deren Umgebung in der Kamera 40 aufgenommen wird. Das so entstehende Bild mit Fovea und Fixierlicht-Spot kann dem Arzt auf einem Bildschirm angezeigt werden, damit er die Fixierung seitens des Patienten überprüfen kann. Die Auswertung der Bilder kann auch im Rechner 38 mit den Techniken der Bildverarbeitung automatisiert werden.

In Figur 1 sind die einzelnen Laser-Strahlungsquellen und die Umlenkspiegel nur schematisch im Sinne guter Übersichtlichkeit dargestellt. In Praxis wird man den Excimer-Laserstrahl anders als dargestellt einkoppeln, insbesondere möglichst nahe am Auge, da an teildurchlässige Spiegel für UV-Strahlung besondere Anforderungen gestellt werden. Somit wird in Praxis die Anordnung der teildurchlässigen Spiegel so sein, daß der Spiegel UV-S noch unter dem Spiegel 41 liegt.

Gemäß einer bevorzugten Ausgestaltung der Erfindung werden die in Figur 1 gezeigten Kameras 36 und 40 (die Kamera 36 zusätzlich zu der oben beschriebenen Funktion) so verwendet, daß mit der Kamera 36 ein Bild der Pupille 18 aufgenommen und in den Rechner 38 eingegeben wird, während mit der Kamera 40 das oben beschriebene Bild in der Ebene der Fovea 30 mit dem Fixierlicht-Spot auf der Netzhaut aufgenommen wird und ebenfalls in den Rechner 38 eingegeben wird. Beide Bilder werden in bezug auf eine feste gemeinsame Achse aufgenommen, so daß beide Bilder im Rechner 38 einander überlagert werden können, so daß eine Aussage möglich ist über die relative Positionierung von Fovea, Fixierlicht-Spot und Pupille.

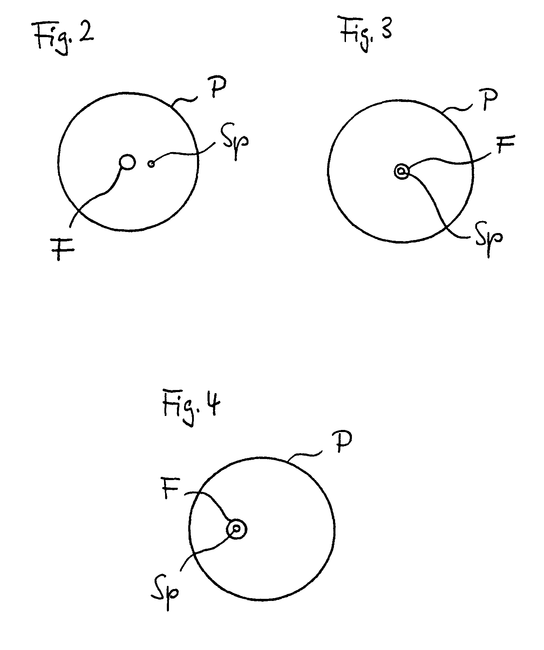

Dies ist schematisch in den Figuren 2, 3 und 4 dargestellt. Diese Figuren zeigen jeweils die angesprochenen Überlagerungsbilder.

Figur 2 zeigt den Rand P der Pupille, die Fovea F und den Fixierlicht-Spot Sp, wie es mit Hilfe eines oben beschriebenen Überlagerungsbildes der beiden Kameras 36 und 40 im Rechner 38 erhalten und gegebenenfalls auf einem geeigneten Bildschirm angezeigt werden kann. Bei der Situation gemäß Figur 2 fixiert der Patient die Fixierlichtquelle nicht korrekt. Dies erkennt der Arzt daran, daß der Fixierlicht-Spot Sp nicht genau in der Fovea F liegt, sondern dazu versetzt ist. Dies hat zur Folge, daß der Arzt geeignete Maßnahmen ergreifen muß, um den Patienten zu einer genauen Fixierung zu veranlassen.

Figur 3 zeigt den Idealfall, bei dem der Patient genau fixiert und somit der Fixierlicht-Spot Sp genau konzentrisch mit der Fovea F liegt. Bei dem Befund gemäß Figur 3 ist die Fovea F auch zentrisch in der Pupille P. Dies muß nicht immer der Fall sein. Figur 4 zeigt ein Beispiel, bei dem der Patient zwar die Fixierlichtquelle 22 korrekt fixiert, so daß der Fixierlicht-Spot Sp genau in der Fovea F liegt, jedoch liegt die Fovea F nicht zentrisch in der Pupille P. Es sind noch kompliziertere Befunde möglich, bei denen der dargestellte Pupillenrand keineswegs die in den Figuren dargestellte ideale Kreisform hat, sondern davon abweicht. Dies gilt insbesondere nach bereits früher durchgeführten ophtalmologischen Operationen.

Bei einem Befund gemäß Figur 4 kann der als exzentrisch in bezug auf die Pupille P gefundene Ort von Fixierlicht-Spot Sp und Fovea F als Ablationszentrum gewählt werden, d. h. die Ablation erfolgt zentriert in bezug auf den Mittelpunkt von Sp und F. Diese Wahl hat auch zu guten Ergebnissen geführt.

## Patentansprüche

1. Vorrichtung für eine ophtalmologische Augenbehandlung mit
- einem Behandlungslaser gerät zum Abgeben eines Behandlungslaserstrahls (UV) zum Ablatieren von Teilen der Kornea (12) und
- einer Fixierlichtquelle 22 zum Abgeben eines Fixierlichtstrahls (24), der vorgesehen ist, um vom Patienten fixiert zu werden,
**gekennzeichnet durch**
- eine Kamera (40) zum Abbilden eines vom Fixierlichtstrahl (24) auf der Netzhaut, insbesondere der Fovea, erzeugten Lichtflecks (Sp) und der Fovea (F).

2. Vorrichtung nach Anspruch 1,
**gekennzeichnet durch**
eine Einrichtung (36) zum Abbilden der Pupille (18, P) derart, daß das Bild des Lichtflecks (Sp) des Fixierlichtstrahls (24) und das Bild (P) der Pupille überlagert sind.

3. Vorrichtung nach Anspruch 2,
**dadurch gekennzeichnet, daß**
die Einrichtung zum Abbilden der Pupille die genannte Kamera ist.

4. Vorrichtung nach Anspruch 2,
**dadurch gekennzeichnet, daß**
die Einrichtung zum Abbilden der Pupille eine zweite Kamera (40) ist.

5. Vorrichtung nach Anspruch 4,
**dadurch gekennzeichnet, daß**
die zweite Kamera (40) auch einem Eye-Tracker zugeordnet ist.

## Claims

1. Apparatus for an ophthalmologic eye treatment comprising:
- a therapeutic laser device for emitting a therapeutic laser beam (UV) for ablating parts of the cornea (12), and
- a fixation-light source (22) for emitting a fixation-light beam (24), which is provided so that the patient may fix his/her gaze upon it,
**characterized by**
- a camera (40) for imaging a luminous spot (Sp), created by the fixation-light beam (24) on the retina, especially the fovea, and the fovea (F).

2. Apparatus according to claim 1,
**characterized by** a means (36) for imaging the pupil (18, P) in such a manner that the image of the luminous spot (Sp) of the fixation-light beam (24) and the image (P) of the pupil are superimposed.

3. Apparatus according to claim 2,
**characterized in that** the means for imaging the pupil is said camera.

4. Apparatus according to claim 2,
**characterized in that** the means for imaging the pupil is a second camera (40).

5. Apparatus according claim 4,
**characterized in that** the second camera (40) is also associated to an eye tracker.

## Revendications

1. Dispositif pour un traitement ophtalmologique des yeux, avec un instrument de traitement au laser, émettant un faisceau laser de traitement (UV) pour l'ablation de parties de la cornée (12) et avec une source lumineuse à fixer (22) émettant un faisceau lumineux à fixer (24) destiné à être fixé par le patient, **caractérisé par** une caméra (40) pour la reproduction d'un point lumineux (Sp) généré sur la rétine, notamment sur la fovéa, par le faisceau lumineux à fixer (24) et de la fovéa (F).

2. Dispositif selon la revendication 1, **caractérisé par** un système (36) pour la reproduction de la pupille (18, P) de façon à ce que l'image du point lumineux (Sp) du faisceau lumineux à fixer (24) et l'image (P) de la pupille soient superposées.

3. Dispositif selon la revendication 2, **caractérisé en ce que** le système de reproduction de la pupille est la caméra citée.

4. Dispositif selon la revendication 2, **caractérisé en ce que** le système pour la reproduction de la pupille est une deuxième caméra (40).

5. Dispositif selon la revendication 4, **caractérisé en ce que** la deuxième caméra (40) est également associée à un Eye-Tracker.
